# EUROPEAN PATENT APPLICATION

(11) **EP 2 896 377 A1**
(43) Date of publication of application: **22.07.2015**
(21) Application number: 14382010.8
(22) Date of filing: 15.01.2014
(51) Int. Cl.: A61B 17/42, D05B 91/04, A41D 19/00, A61B 17/00, A61B 19/04

(54) **Birth assisting device**

(71) Applicant: Haimovich Segal, Sergio, 08922 Santa Coloma de Gramanet, Barcelona (ES)
(72) Inventor: Haimovich Segal, Sergio, 08922 Santa Coloma de Gramanet, Barcelona (ES); Cassou Arnaiz, Lluis, 08330 Premià de Mar, Barcelona (ES)
(74) Representative: Gallego Jiménez, José Fernando

(57) **Abstract**

Birth assisting devices are disclosed. The devices may comprise a compartment comprising a front side, a back side, and two lateral sides, which is open from a bottom side and configured to host one or more fingers, wherein a first area of the compartment has a first stiffness and a second area has a second higher stiffness, and wherein the second area comprises at least a portion of one of the lateral sides of the compartment. The device may allow a birth practitioner, such as a physician or a midwife, to rotate the head of a fetus during labor avoiding the use of a forceps or vacuum. Furthermore, it may protect the fingers of the practitioner from lesions.

## Description

The present disclosure relates to gynaecological or obstetrical instruments and more specifically to birth assisting devices.

### BACKGROUND ART

There are many situations during childbirth where the fetus, and more particularly the head of the fetus, is not positioned appropriately for passage through the birth canal. In these cases, and assuming a vaginal birth is desired, the qualified childbirth practitioner, a physician or a midwife, needs to manually rotate the head of the fetus. If this fails, then a C-section operation may be inevitable to perform delivery of the baby.

The rotation of the head may be performed either by the practitioner's fingers or with the use of appropriate instruments. Such instruments may be forceps or vacuum extractors. In child deliveries that involve forceps or vacuum extractors, the baby may present light hematomas that normally disappear a few days after they are born. The danger that the child may present more serious problems is relatively low. However, during the more recent years, the use of such instruments has decreased, mostly because of the rising importance that is given to the health of the newborn. Furthermore, these instruments are perceived negatively by the majority of the parents and the mere possibility of their use may cause fear and stress during delivery. Even if there is no fear involved, there is a danger to cause a rupture in the perinea which in turn is associated with fecal and gas incontinence in women.

On the other hand, the percentage of childbirths that are performed with a C-section operation has been increasing in recent years. The C-section rate has risen to 46% in China and is over 25% in many Asian, European and Latin American countries. A number of these childbirths may possibly have been performed vaginally had the head of the fetus been in the appropriate position for entry in the birth canal.

A number of birth practitioners may try to use their fingers, namely the index and middle finger, in an attempt to rotate the head and position the head appropriately for birth. Although this practice may have a desirable effect in some cases, this is not always possible and it may require significant force from the part of the practitioner. This force may hurt the joints of the fingers of the practitioner.

It would be desirable to have a child assisting device that at least partially resolves the aforementioned problems.

### SUMMARY OF THE INVENTION

In a first aspect, a birth assisting device is disclosed. The device has a compartment comprising a front side, a back side, and two lateral sides, which is open from the bottom side and configured to host at least one finger. A first area of the compartment has a first stiffness and a second area has a second higher stiffness. The second area comprises at least a portion of one of the lateral sides of the compartment.

Throughout the document, the term "front side" is used to indicate a side of the device that is intended to be in contact with the (head of the) fetus when the device is in use. Seen from another perspective, the "front side" may be considered to indicate the side that corresponds to the palm side of the fingers when the device is worn. Accordingly, the term "back side" is used to indicate a side of the device that is not likely to come in contact with the fetus, i.e. the side that corresponds to the back of the fingers when the device is worn. Likewise, the term "lateral sides" refers to the sides that connect the front side with the back side.

The dual stiffness device achieves two purposes: the area with the first stiffness may allow certain flexibility so that the head of the fetus may be embraced in a smooth way while the practitioner has more cutaneous control of the process; on the other hand, the area with the second stiffness may protect the joints of the finger(s) of the practitioner when performing a rotation function. The main advantage of the birth assisting device is that it may avert an invasive operation, such as a C-section, that is associated with a higher morbidity rate compared to non-invasive techniques. Furthermore it facilitates the childbirth process since the application of a forceps or a vacuum includes anesthesia and episiotomy that are avoided with the use of the proposed device.

In some embodiments, the second area of increased stiffness may include at least a portion of both of the lateral sides of the compartment. In that case the fingers of the practitioner may be protected in either rotation direction. The proposed device can facilitate the work of the practitioner, diminish the forces at the joints and allow for a better grip. It may strengthen the structure of the metacarpal joints while allowing anteroposterior flexibility. Thus, it may be used as an intermediate tool between the use of bare fingers and the use of forceps. It may, therefore, substitute the use of forceps in certain cases as it allows rotation forces greater than the ones exerted with bare fingers. The different stiffness could be achieved either by the same material having different density, or by the same material having different thickness or by using a different material altogether.

In some embodiments, the front side may be configured to adhere to the skin of a fetus. This may allow a grip that resembles the grip of the fingers or may allow for a firmer grip. As a result, the practitioner shall excise a force to the head that may be similar or lower to the force that he would excise without using the device with similar effects.

In some embodiments, a coefficient of friction between at least one portion of the front side and the skin (head) of the fetus may be lower in a first direction than in a second direction. The first direction may be a direction of entry into the birth canal and the second direction may be a direction of rotation of the head of the fetus. The advantage is that the lower coefficient of friction in the one direction allows a smooth introduction of the device without causing any distress and without irritating the birth canal. On the other hand, the higher coefficient of friction in the other direction allows for a better adherence to the skin of the baby when the rotation is performed, taking into consideration the humid environment of the uterus and of the birth canal. To achieve the different coefficients of friction in the two directions, the surface of (a portion of) the front side may have different geometric properties in the first direction than in the second direction thus having different contact surfaces in one direction from the other.

In some embodiments a portion of the front side is raised to adhere to the skin of the baby. The portion may be raised with respect to the rest of the front side so that a partially concave surface may be formed to best fit the convex surface of the baby's head.

In some embodiments, at least a portion of the front area may comprise a plurality of protrusions. These may be uniformly spaced and each of the protrusions may result in a first coefficient of friction between the front side and the fetus in a first direction and a second coefficient of friction in a second direction. In one example this is achieved by protrusions in the shape of circular sections or arcs. The arcs or circular sections may be arranged in a direction parallel to the axis of the fingers. The coefficient of friction in this direction (corresponding to a direction of entry of the device in the birth canal) may thus be lower than the coefficient of friction in a direction substantially perpendicular to the axis of the fingers (the direction of rotation of the head of the fetus).

In some embodiments, at least a portion of the lateral sides may be thicker than the first area. The advantage of such a configuration is that the device may be formed of the same material having a uniform density. As at least a portion of the lateral sides are thicker than the first area, this may have the effect that said at least one portion of lateral sides presents a stiffness that may be higher than that of the first area.

In some embodiments, the at least one of the lateral sides may also comprise a portion that is more flexible than the rest of said lateral side to allow flexing of the fingers. The flexing of the fingers may allow a better grip of the baby's head and a more natural feel for the practitioner.

In some embodiments the flexible portion may be located at an area of the lateral side that substantially corresponds to the joint between the proximal phalanx and the intermediate phalanx of a finger, when the birth assisting device accommodates said at least one finger. As the fingers may only bend at distinct points, this has the advantage that the device may only bend when the practitioner forces it to do so and not accidentally by other forces. The flexible portion may either be substantially at the joint between the proximal phalanx and the intermediate phalanx or may incorporate an area corresponding to a portion or the whole of the intermediate phalanx. Furthermore, the less flexible portion may be interrupted by the flexible portion or it may be continuous with a wider non-flexible area towards the portions that correspond to the metacarpal and the distal phalanges of the finger and a reduced area around the joint between the proximal phalanx and the intermediate phalanx of the finger. The less flexible area may be in the shape of an hourglass or even a lemniscate.

In some embodiments the back side may comprise creases to allow adaptation of the device to the fingers. As a result, the same device may be worn by practitioners that may have a different finger thickness.

In some embodiments the device may further comprise a flange to allow easy extraction of the device. As the entire device may be inserted in the birth canal, the flange may assure that the device shall not accidentally remain in the birth canal when the practitioner retracts.

In some embodiments the compartment may comprise a first section to host a middle finger and a second section to host an index finger. As the two fingers have distinct anatomy, having different sections for each finger allows sizing each compartment accordingly.

In some embodiments, the flange may be located between the first section and the second section. Therefore the device may be easily removed if the practitioner retracts the device by pulling on the flange, e.g. by keeping the two fingers pressed together.

In some embodiments, the first section of the compartment may be longer than the second section of the compartment to account for the difference in length of the index finger and the middle finger. This difference in length may also allow the practitioner to easily identify the front and back side of the device.

In any of the embodiments herein described, the compartment may be adapted to conform to the at least one finger to allow sensitivity to touch. In one scenario the material of the device is such that it allows to conform to the finger(s) and in another scenario the device is designed to follow the natural curvature of the finger(s) of the practitioner. For purposes of reducing the complexity in manufacture, the device may be produced in different sizes to allow conformity to different size of fingers. Furthermore, a version for right handed practitioners and a version for left handed practitioners is envisaged.

In some embodiments, the device may be made of polymeric material. Any polymeric material that is considered appropriate for entry in the birth canal may be used.. The material used may be easily sterilized, for example by ethylene oxide.

The material used may allow the device to be of a disposable nature. Therefore it may be used in areas where the sanitary conditions do not allow for a proper C-section or where it may be difficult or impossible to sterilize forceps or other traditional instruments. Furthermore, the device may be produced economically so that it may be more accessible to practitioners than any other similar purpose device. Additional objects, advantages and features of embodiments of the invention will become apparent to those skilled in the art upon examination of the description, or may be learned by practice of the invention.

### BRIEF DESCRIPTION OF THE DRAWINGS

Particular embodiments of the present invention will be described in the following by way of non-limiting examples, with reference to the appended drawings, in which:
Figure 1 is a front perspective view of a birth assisting device according to an embodiment;
Figure 2 shows a rear perspective view of a birth assisting device according to an embodiment;
Figure 3 is a bottom view of a birth assisting device according to another embodiment.

### DETAILED DESCRIPTION OF EMBODIMENTS

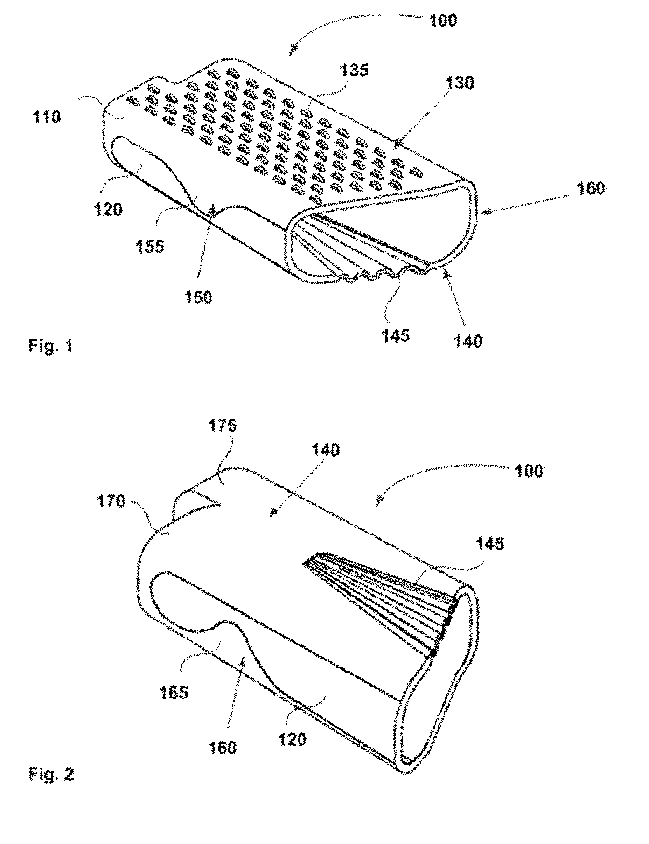

Figures 1 and 2 are perspective front and rear views of a birth assisting device according to an embodiment. The device shown is arranged to accommodate two fingers and more precisely the index finger and the middle finger of a right handed practitioner.

The birth assisting device 100 comprises a compartment that has a first area 110 that has a first stiffness and a second area 120 that has a second stiffness. The second stiffness is higher than the first stiffness.

The first area 110 may include the front side 130, the back side 140 and portions of the lateral sides 150 and 160. The second area 120 may include a portion of each of the lateral sides 150 and 160. As may be seen in Fig. 1, the second area may extend along the lateral sides and may have a first section that is wider than a second section. The second section of the second area 120 is arranged in an area 155 of the second side that substantially corresponds to the position of the joint between the proximal phalanx and the intermediate phalanx of the finger when the device is worn by a practitioner. The second area 120 is such as to allow the natural bending of the fingers. At the same time, the second area 120 is stiff enough so as to not allow any harm to the finger joints caused by the forces exerted when rotating the head of the baby.

In this particular example, a local reduction in width of the area of increased stiffness 120 (i.e. a notch) corresponds substantially to the position of the joint between the proximal phalanx and the intermediate phalanx of the finger when the device is worn by a practitioner. From this local reduction in width towards the tip of the fingers, the second area of increased stiffness widens.

The front side 130 is shown pebbled with protrusions 135. Each of the protrusions is shown to be arc shaped. The arcs may be arranged in a direction substantially parallel to the axis of the fingers. This shape and orientation has the advantage that the pebbled area has a resulting different friction -lower- in the direction that is substantially parallel to the axis of the fingers than in the direction that is substantially perpendicular to the axis of the fingers where the roughness is higher. This is caused by the reduced area of sliding contact in one direction than in the other.

The reason for such an arrangement is that the direction substantially parallel to the axis of the fingers corresponds to the direction that the device will follow when entering and exiting the birth canal. It is therefore desirable to reduce friction during insertion and removal that may cause irritation to the birth canal. At the same time, it is important that the device adheres to the baby's head when the practitioner has gripped the head in the birth canal. Once the head is gripped, any rotation will take place in the direction that is substantially perpendicular to the axis of the fingers, thus the roughness may be high enough to make sure that the head does not slip, and at the same time not high enough that it could irritate the head.

The arc shape of the protrusions 135 allows for such a dual roughness or dual friction arrangement. One skilled in the art may appreciate that other protrusion arrangements may have the same effect. For example, the front side may be creased from one side to the other, so that the protrusions may be in the form of lines parallel to the axis of the fingers.

A portion of the back side 140 of the device 100 is shown with creases 145. The creases 145 are best shown in Fig. 2. The creases allow for practitioners having different size -width- of fingers to use the same device. The device is adaptable to the fingers. The creased area is wider near the opening of the device (bottom of the compartment) and converges in an area substantially around the centre of the back side 140. Alternatively, instead of a creased portion, a single slit at the back of the device could perform a similar function, although that would leave a portion of the fingers uncovered. A practitioner would normally wear a glove, such as a latex glove, before using the device. Therefore, even if a portion of the fingers is not covered by the device, the fingers will nevertheless be protected by the glove.

Furthermore, the device shown in fig. 1 and 2 has a first sub-compartment 170 to host an index finger of a right-handed person and a second sub-compartment 175 to host a middle finger thereof. The inner portions of each of the sub-compartments 170, 175 may be padded to account for differences in the length of fingers between practitioners.

Alternatively, the whole device may be incorporated in a glove arrangement. The birth assisting device would then comprise the sheaths of the index and middle fingers and the rest of the glove would be made of any appropriate material such as latex. The birth assisting device could either have a single compartment for the two fingers or one compartment for each finger. In the latter case, the area between the index finger and the middle finger may optionally be made of the same material as the rest of the glove (e.g. latex) so that certain flexibility is allowed in the movement of the fingers. This may be particularly useful when the practitioner is inciting the dilation process.

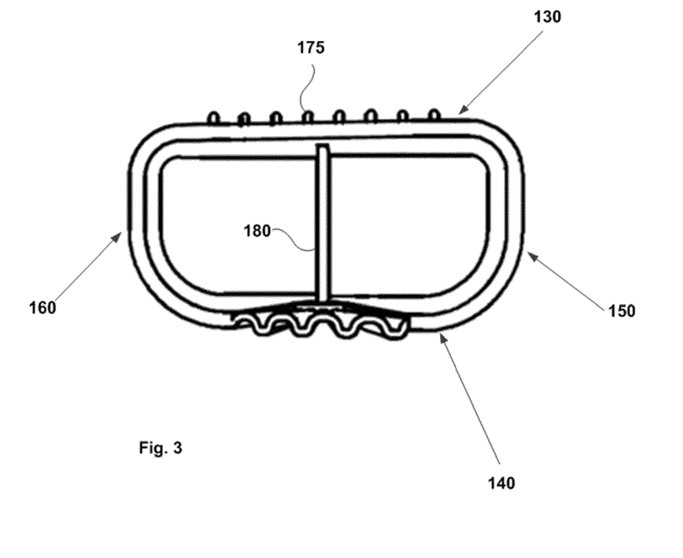

Figure 3 shows a back side of a birth assisting device according to another example. The device has a flange 180 separating the two sub-compartments that are arranged to accommodate the index and middle finger. A set of protrusions 175 is shown on the front side 130 of the device. The protrusions 175 shown in this example may correspond to the protrusions shown in the embodiment of figures 1 and 2. Accordingly, the back side 140 is creased to allow adaptation of the device to dissimilar fingers.

During a labor process, a birth practitioner may determine that the head of the baby is not in a desired position that would be suitable for vaginal birth. In other cases, the practitioner may simply want to accelerate the rotation of the head.

The practitioner would position the index and middle finger in a birth assisting device such as one of the devices described with reference to Fig. 1-3. Then, the practitioner would introduce the device in the birth canal until he reaches the head of the baby. The practitioner would then flex his or her fingers and bend the device so that it may grip a portion of the baby's head. Then, based on the orientation of the head, he may exert a rotational force to the head in order to rotate the head to a desired position that may be suitable for vaginal birth.

It is understood that the dual stiffness of the device allows flexing of the fingers while protecting the metacarpal joints when certain force is used to rotate the head. Once the head of the baby is in a desired place, the practitioner would release the head and remove the hand from the birth canal.

If necessary, in order for the device to be removed from the birth canal together with the hand, the practitioner may e.g. press the two fingers against the lateral sides. Alternatively, if provided, the practitioner may firmly grip a flange, such as flange 180. In case the flange is between the two finger sub-compartments, such as in Fig. 3, the practitioner need only grip firmly the flange with the two fingers to perform the extraction of the device.

The device may be produced in different sizes to account to different finger sizes of the practitioners. Furthermore, the device may have right-handed versions or left-handed versions so that practitioners of any handedness may use them. It is perceived that the device would be produced as a disposable device. However, it is also perceived that the device may be reusable if appropriately sterilised after each use. The decision to dispose of or reuse the device may be a factor of cost, material and/or availability of stock.

Any of the embodiments disclosed herein may be made from suitable materials. In some examples, a polymeric material may be used.

Although only a number of particular embodiments and examples of the invention have been disclosed herein, it will be understood by those skilled in the art that other alternative embodiments and/or uses of the invention and obvious modifications and equivalents thereof are possible. Furthermore, the present invention covers all possible combinations of the particular embodiments described. Thus, the scope of the present invention should not be limited by particular embodiments, but should be determined only by a fair reading of the claims that follow.

## Claims

1. A birth assisting device comprising:
a compartment comprising a front side, a back side, and two lateral sides, which is open from a bottom side and configured to host one or more fingers, wherein a first area of the compartment has a first stiffness and a second area has a second higher stiffness, and wherein the second area comprises at least a portion of one of the lateral sides of the compartment.

2. The birth assisting device according to claim 1, wherein the second area comprises at least a portion of both of the lateral sides of the compartment.

3. The birth assisting device according to claim 2, wherein at least a portion of the front side of the compartment is configured to adhere to the skin of a fetus.

4. The birth assisting device according to claim 3, wherein a coefficient of friction between said at least one portion of the front side and the skin of the fetus is lower in a first direction than in a second direction, the first direction being a direction of entry and the second direction being a direction of rotation.

5. The birth assisting device according to any of claims 3 or 4, wherein a portion of the front side of the compartment is raised to adhere to the skin of the baby.

6. The birth assisting device according to any of claims 3-5, wherein the front area comprises a plurality of protrusions.

7. The birth assisting device according to any of the previous claims, wherein the second area of the compartment has an increased thickness with respect to the first area.

8. The birth assisting device according to any of the previous claims, wherein at least one of the lateral sides comprises a portion of the second stiffness and a portion that is more flexible than the rest of said lateral side to allow flexing of the fingers.

9. The birth assisting device according to claim 8, wherein said flexible portion is located at an area of said lateral side that in use corresponds to a joint between a proximal phalanx and an intermediate phalanx of a finger, when the birth assisting device accommodates said one or more fingers.

10. The birth assisting device according to any of the previous claims, wherein the back side comprises creases to allow adaptation of the compartment to the fingers.

11. The birth assisting device according to any of the previous claims, further comprising a flange to allow easy extraction of the device.

12. The birth assisting device according to any of the previous claims, wherein the compartment is configured to host exactly two fingers.

13. The birth assisting device according to any of the previous claims, wherein the compartment comprises a first section to host a middle finger and a second section to host an index finger.

14. The birth assisting device according to claim 13, wherein a flange is located between the first section and the second section.

15. The birth assisting device according to claim 13 or 14, wherein the first section of the compartment is longer than the second section of the compartment to account for the difference in length of the index finger and the middle finger.
